# EUROPÄISCHE PATENTSCHRIFT

(11) **EP 1 234 592 B1**
(45) Veröffentlichungstag und Bekanntmachung des Hinweises auf die Patenterteilung: **01.09.2004**
(21) Anmeldenummer: 01130832.7
(22) Anmeldetag: 27.12.2001
(51) Int. Cl.: A61M 16/00, A61M 16/10, A61M 16/08

(54) **Verfahren und Vorrichtung zur Schalldämmung bei Beatmungsgeräten**
Method and device for noise reduction in ventilation systems
Procédure et appareil pour réduire le bruit produit par les appareils de ventilation

(30) Priorität: 19.02.2001 DE 10107990
(43) Veröffentlichungstag der Anmeldung: 28.08.2002
(60) Teilanmeldung: 04009173.8
(73) Patentinhaber: Weinmann Geräte für Medizin GmbH & Co. KG, 22525 Hamburg (DE)
(72) Erfinder: Feldhahn, Karl-Andreas, 22761 Hamburg (DE); Paesch, Rainer, 25451 Quickborn (DE)
(74) Vertreter: Klickow, Hans-Henning, Dr.-Ing.

(56) Entgegenhaltungen:
- EP-A- 0 512 285
- DE-A- 19 731 355
- JP-A- 2000 281 315

## Beschreibung

Die Erfindung betrifft ein Verfahren zur Schalldämmung bei der Beatmung eines Patienten, bei dem mindestens ein Teil des Atemgases durch eine Dämpfungsbox hindurchgeleitet wird, die mindestens einen Strömungskanal aufweist, in dessen Bereich die Atemluft an einem schalldämmenden Material vorbeigeführt wird.

Die Erfindung betrifft darüber hinaus eine Vorrichtung zur Schalldämmung bei der Beatmung eines Patienten, die als eine Dämpfungsbox ausgebildet ist, die einen von einem Gehäuse umschlossenen Innenraum aufweist, der mit mindestens einem Atemgaseinlaß und mit mindestens einem Atemgasauslaß versehen ist und in der mindestens bereichsweise ein schalldämmendes Material zur Begrenzung mindestens eines Strömungskanals eingesetzt ist.

Ein derartiges Verfahren und eine derartige Vorrichtung werden beispielsweise in der DE-OS 197 31 355 beschrieben. Der Innenraum der Dämpfungsbox wird dabei durch Strömungsleitelemente unterteilt, um einen im Hinblick auf die Geräuschdämmung optimierten Strömungsweg bereitzustellen. Gemäß dem Stand der Technik ist es üblich, in ein metallisches Gehäuse der Dämpfungsbox zur Verbesserung der Dämpfungswirkung Platten aus Schaumstoff einzukleben.

Die bekannten Vorrichtungen werden üblicherweise ausschließlich individuell für einen bestimmten Patienten genutzt, da sich eine Desinfektion aufgrund der vorliegenden Bauart schwierig gestaltet beziehungsweise nicht durchführbar ist. Die bekannten Konstruktionen führen ebenfalls dazu, daß auch bei einer Individualanwendung die Betriebsdauer durch eine zunehmende Anreicherung von Keimen und Bakterien im Bereich des Schaumstoffmaterials begrenzt ist.

Aus der EP-A 0 512 285 ist es bekannt, bei der Beatmung eines Patienten von einem Mikroprozessor gesteuerte Ventile im Zusammenhang mit einer Befeuchtungseinrichtung einzusetzen. Die Ventile sowie ein Teil des pneumatischen Leitungssystems werden in eine sogenannte Patientenbox eingebaut.

Aufgabe der vorliegenden Erfindung ist es daher, ein Verfahren der einleitend genannten Art derart zu verbessern, daß sowohl eine erhöhte Nutzungsflexibilität als auch eine verlängerte Betriebsdauer erreicht werden. Diese Aufgabe wird erfindungsgemäß dadurch gelöst, daß das schalldämmende Material nach einer vorgebbaren Betriebsdauer aus der Dämpfungsbox entnommen wird, daß anschließend eine Desinfektion eines Gehäuses der Dämpfungsbox durchgeführt wird und daß in die Dämpfungsbox ein keimfreies schalldämmendes Material zur Wiederherstellung der Betriebsfähigkeit eingesetzt wird.

Weitere Aufgabe der vorliegenden Erfindung ist es eine Vorrichtung der einleitend genannten Art derart zu konstruieren, daß verbesserte Eigenschaften im Hinblick auf eine Wiederverwendbarkeit erreicht werden.

Diese Aufgabe wird erfindungsgemäß dadurch gelöst, daß das schalldämmende Material plattenartig gestaltet und lose in das Gehäuse eingesetzt ist und daß das schalldämmende Material durch elastische Verspannungen innerhalb des Gehäuses fixiert ist sowie daß das schalldämmende Material auf einen plattenartig gestalteten Träger aufgebracht ist.

Durch die lose Anordnung des schalldämmenden Materials ist es möglich, eine Desinfektion des Gehäuses separat und somit im Hinblick auf die jeweiligen Materialeigenschaften optimiert durchzuführen. Aufgrund dieser materialspezifischen Desinfektion ist es möglich, eine Wiederverwendbarkeit der Vorrichtung zu erreichen. Ebenfalls ist es möglich, bei nachlassenden elastischen Eigenschaften des schalldämmenden Materials lediglich das plattenartige lose schalldämmende Material durch neue Bauteile zu ersetzen und das in der Regel aus Metall bestehende Gerätegehäuse weiter zu verwenden. Eine derartige Verfahrensweise hat sowohl unter wirtschaftlichen als auch unter ökologischen Gesichtspunkten eine hohe Bedeutung. Durch die elastische Verspannung des schalldämmenden Materials innerhalb des Gehäuses werden separate Befestigungselemente vermieden, die zu einer erschwerten Montage sowie zu Komplikationen bei der Desinfektion führen könnten.

Unter Desinfektion werden hierbei auch eine Sterilisation oder andere Maßnahmen verstanden, die dafür geeignet sind, eine ausreichende Keimfreiheit zu erreichen.

Durch die erfindungsgemäße Vorrichtung sowie das erfindungsgemäße Verfahren können somit ökonomische und ökologische Vorteile miteinander kombiniert werden, darüber hinaus wird auch eine äußerst einfache Montierbarkeit sowie Demontierbarkeit erreicht. Unter Berücksichtigung der deutlich verlängerten Nutzungsdauer der Dämpfungsbox kann hierdurch pro Zeiteinheit der Betriebsfähigkeit eine erhebliche Kostensenkung realisiert werden.

Eine effektive Keimabtötung kann dadurch erfolgen, daß die Desinfektion mindestens zum Teil thermisch durchgeführt wird.

Bei der Verwendung von wärmeempfindlichen Schalldämmungsmaterialien ist auch daran gedacht, daß die Desinfektion mindestens zum Teil chemisch durchgeführt wird.

Eine andere Desinfektionsvariante beseht darin, daß die Desinfektion mindestens zum Teil durch Strahlungsbeaufschlagung durchgeführt wird.

Eine einfach Montierbarkeit und Demontierbarkeit wird dadurch unterstützt, daß das schalldämmende Material durch Eigenelastizität innerhalb der Dämpfungsbox fixiert wird.

Zur Optimierung einer Strömungsführung wird vorgeschlagen, daß ein Strömungsweg innerhalb der Dämpfungsbox durch herausnehmbare Strömungsleitelemente begrenzt wird.

Eine effektive Schalldämmung von Gebläsegeräuschen kann dadurch erfolgen, daß die Atemgasdurchleitung durch die Dämpfungsbox hindurch durch ein innerhalb der Dämpfungsbox angeordnetes Gebläse unterstützt wird.

Eine einfache Handhabung bei gleichzeitig hoher mechanischer Stabilität kann dadurch erreicht werden, daß das schalldämmende Material auf einen plattenartigen Träger aufgebracht ist.

Eine hohe Dämmwirkung wird dadurch unterstützt, daß das schalldämmende Material aus Schaumstoff ausgebildet ist.

Eine weitere Erleichterung der Handhabung kann dadurch erreicht werden, daß mindestens ein Teil des schalldämmenden Materials als ein Einsatzteil für einen Gehäusedeckel der Dämpfungsbox ausgebildet ist.

Darüber hinaus erweist es sich als vorteilhaft, daß mindestens ein Teil des schalldämmenden Materials als ein Auskleidungsteil für ein Gehäuseunterteil der Dämpfungsbox ausgebildet ist.

Eine einfach Fertigung der verwendeten Bauteile kann dadurch unterstützt werden, daß das Auskleidungsteil einteilig als Faltbauelement mit Falzungen ausgebildet ist.

Eine geringe Anzahl der verwendeten Bauelemente kann dadurch erreicht werden, daß das Auskleidungsteil ein Zentralelement sowie Seitenklappen aufweist.

In der Zeichnung sind Ausführungsbeispiele der Erfindung schematisch dargestellt. Es zeigen:
- Fig. 1: eine Seitenansicht des Gehäuses mit abgenommenem Deckel,
- Fig. 2: eine Seitenansicht des Gehäuses gemäß Blickrichtung II in Fig. 1,
- Fig. 3: eine Draufsicht auf den Deckel,
- Fig. 4: eine Seitenansicht des Deckels gemäß Blickrichtung IV in Fig. 3,
- Fig. 5: eine Seitenansicht des Deckels gemäß Blickrichtung V in Fig.3,
- Fig. 6: eine Darstellung des Schaumstoffeinsatzes für den Deckel,
- Fig. 7: eine Darstellung des als Faltelement ausgebildeten Schaumstoffteils für das Gehäuseunterteil,
- Fig. 8: einen Querschnitt durch das Schaumstoffteil zur Veranschaulichung des Aufbaues aus einem Trägermaterial und einem schalldämmenden Material,
- Fig. 9: eine perspektivische Darstellung eines Strömungsleitelementes zum Einsetzen in die Dämpfungsbox,
- Fig. 10: eine perspektivische Darstellung des Strömungsleitelementes gemäß Fig. 9 bei einer Ansicht von hinten,
- Fig. 11: einen Blick in eine Dämpfungsbox mit abgenommenen Deckel und mit eingesetzten Strömungsleitelementen sowie eingesetztem Motorrahmen für das Gebläse,
- Fig. 12: einen Querschnitt gemäß Schnittlinie XII XII in Fig. 11,
- Fig. 13: eine perspektivische Darstellung des schalldämmenden Materials in Fig. 7 mit hochgeklappten Seitenteilen, um ein Einsetzen in das Gehäuseunterteil zu ermöglichen und
- Fig. 14: eine perspektivische Darstellung des schalldämmenden Materials gemäß Fig. 6.

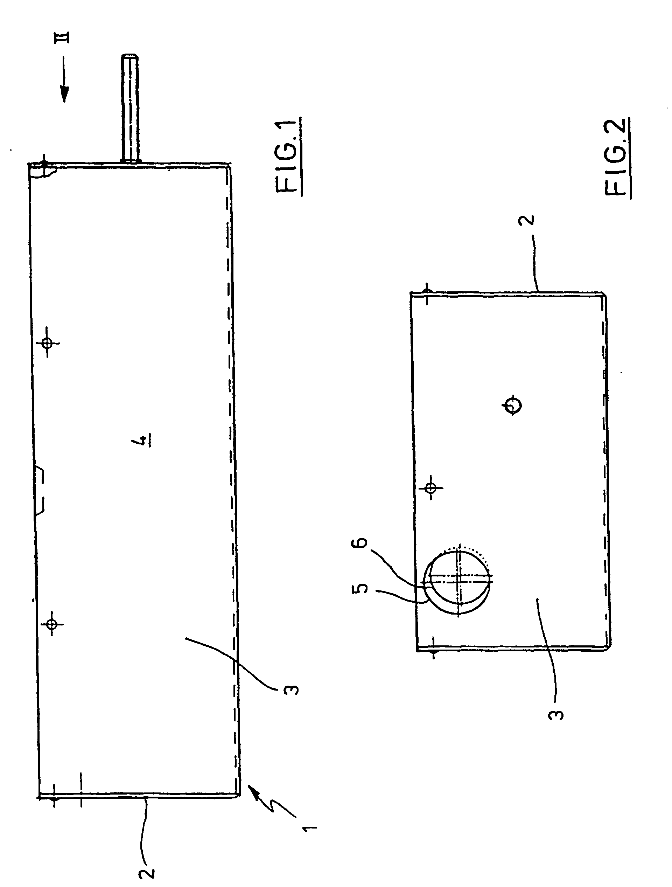

Figur 1 zeigt eine Seitenansicht einer Dämpfungsbox (1), wobei exakt ein Gehäuseunterteil (2) dargestellt ist. Das Gehäuseunterteil (2) weist Wandungen (3) auf, die vorzugsweise aus Metall ausgebildet sind. Ein von der Dämpfungsbox (1) umschlossener Innenraum (4) kann beispielsweise quaderförmig begrenzt sein.

Figur 2 zeigt eine Seitenansicht des Gehäuseunterteils (2) gemäß Figur 1. Es ist erkennbar, daß im Bereich der dargestellten Wandung ein Atemgaseinlaß (5) angeordnet ist. Durch den Innenraum (4) hindurch ist ein Atemgasauslaß (6) erkennbar.

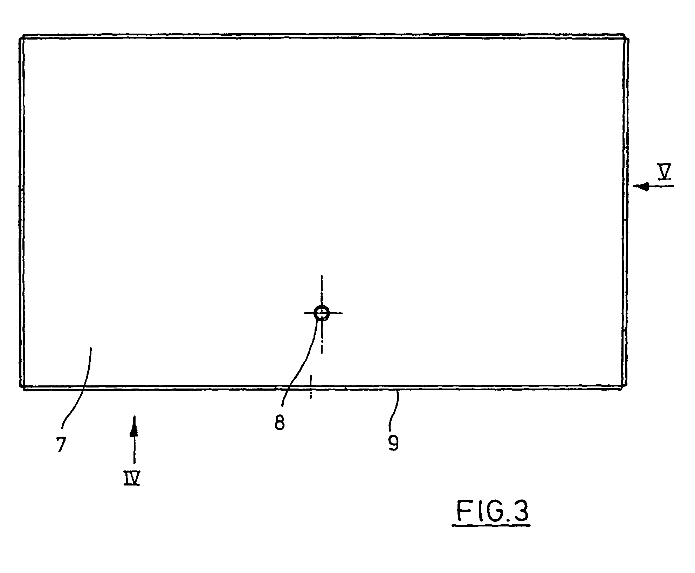

Figur 3 zeigt eine Draufsicht auf einen Gehäusedeckel (7), der auf das Gehäuseunterteil (2) aufsetzbar ist.

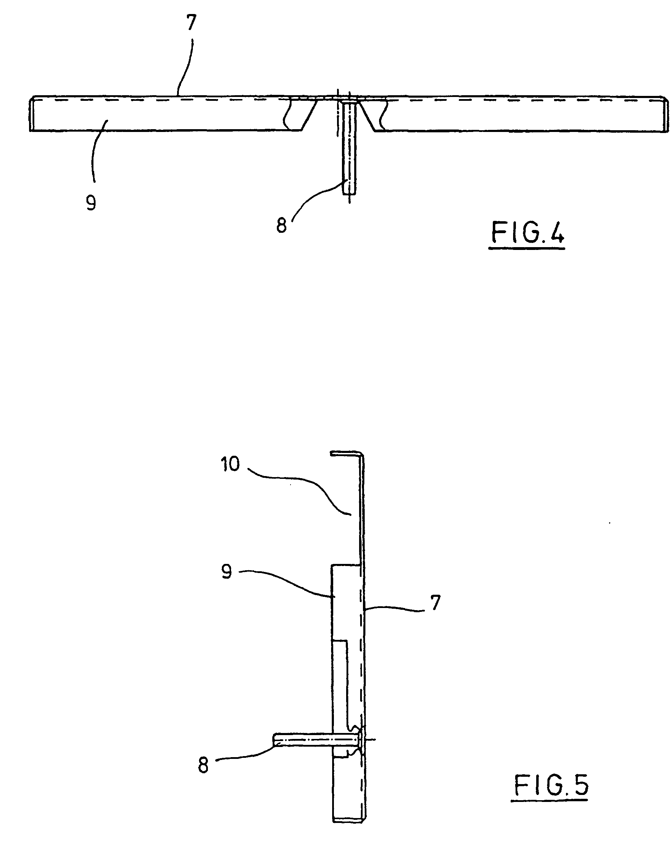

Figur 4 veranschaulicht in einer Seitenansicht die Gestaltung des Gehäusedeckels (7). Insbesondere ist erkennbar, daß der Gehäusedeckel (7) mit einem Halterungsbolzen (8) versehen ist, der für eine Halterung und Fixierung von zusätzlichen Bauteilen im Innenraum (4) dienen kann.

Die Seitenansicht des Gehäusedeckels (7) in Fig. 5 veranschaulicht, daß im Bereich einer seitlichen Deckelwandung (9) eine Ausnehmung (10) angeordnete ist.

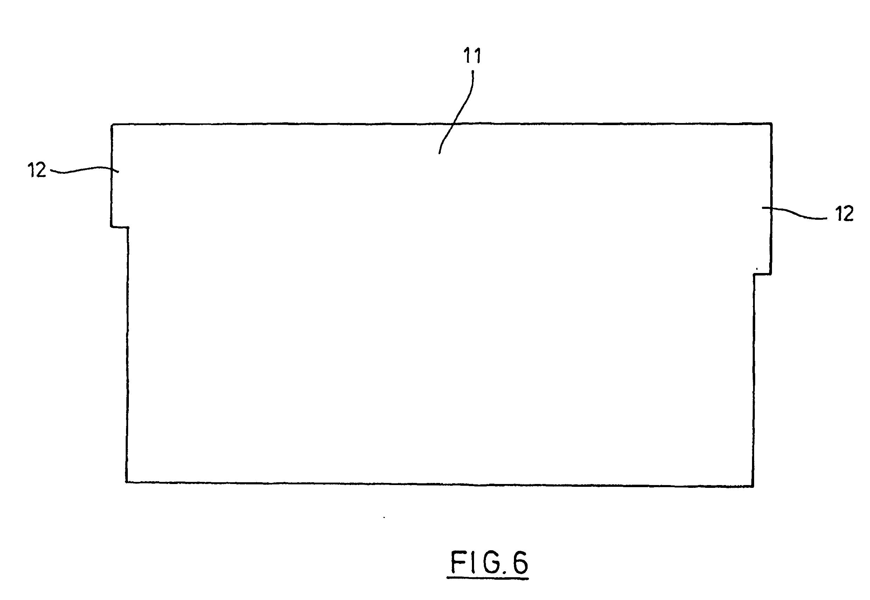

Fig. 6 zeigt ein Einsatzteil (11) aus einem schalldämmenden Material, das derart dimensioniert ist, daß ein Einsetzen in den Gehäusedeckel (7) möglich ist. Das Einsatzteil (11) weist Einsatzlaschen (12) auf , die in die Ausnehmungen (10) der Deckelwandung (9) einführbar sind. Nach einem Einsetzen des Einsatzteiles (11) in den Gehäusedeckel (7) begrenzen die Deckelwandungen (9) seitlich das Einsatzteil (11).

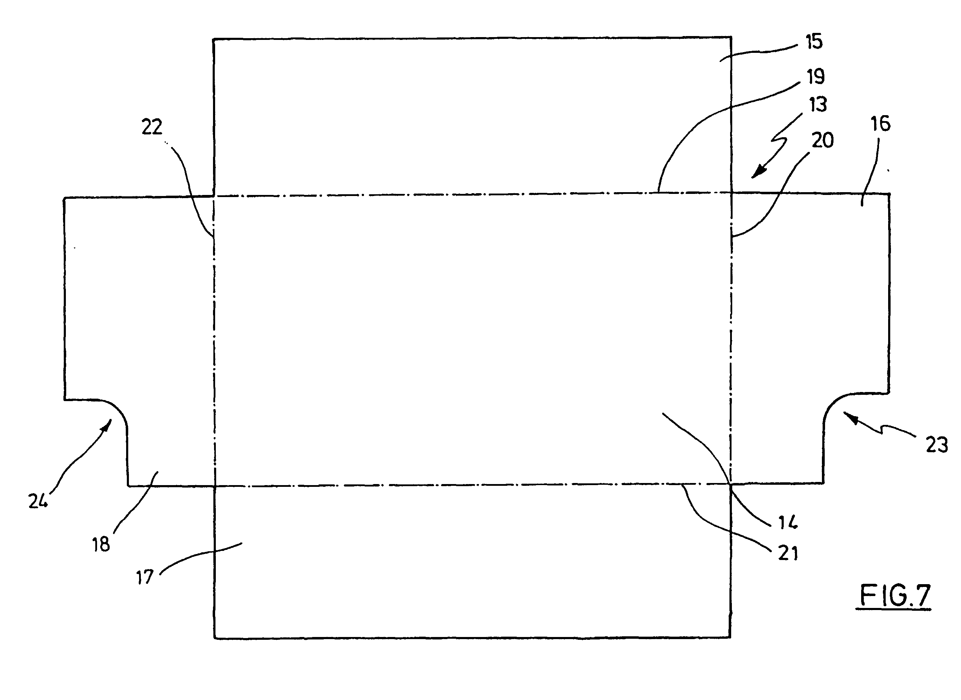

Fig. 7 zeigt ein Auskleidungsteil (13) für das Gehäuseunterteil (2). Das Auskleidungsteil (13) besteht aus einem Zentralelement (14) sowie Seitenklappen (15, 16, 17, 18), die über Falzungen (19, 20, 21, 22) mit dem Zentralelement (14) verbunden sind. zwei der Seitenklappen (15, 16, 17, 18) sind mit Ausnehmungen (23, 24) versehen, die nach einem Einsetzen des Auskleidungsteiles (13) in das Gehäuseunterteil (2) eine Zuleitung und Ableitung von Atemgas durch den Atemgaseinlaß (5) sowie den Atemgasauslaß (6) hindurch ermöglichen.

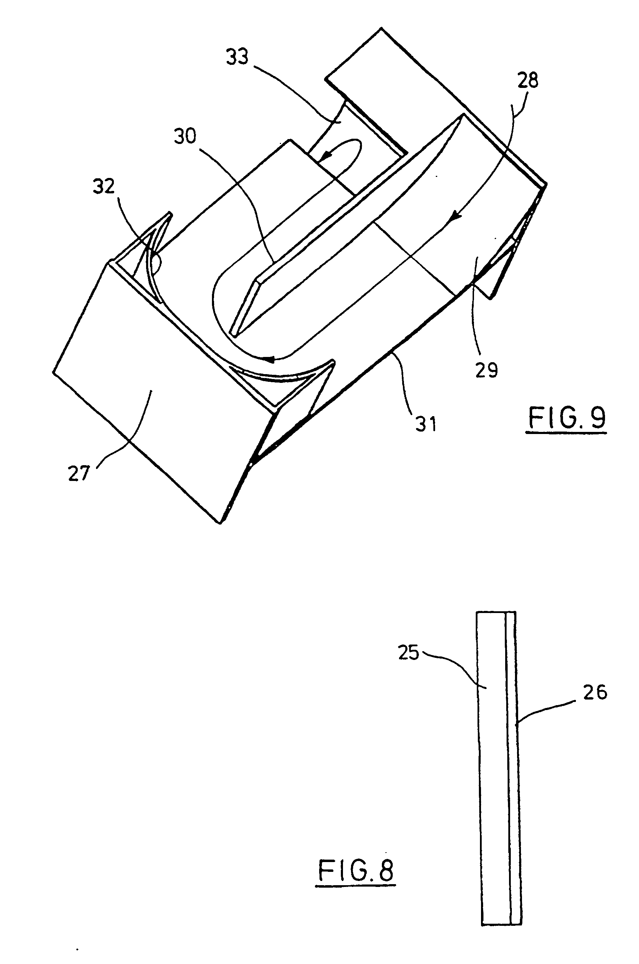

Fig. 8 zeigt eine Seitenansicht des Einsatzteiles (11) beziehungsweise des Auskleidungsteiles (13). Es ist erkennbar, daß ein schalldämmendes Material (25) von einem Träger (26) gehaltert ist. Eine Realisierung kann beispielsweise derart erfolgen, daß der Träger (26) aus Kunststoff, beispielsweise aus Polyethylen ausgebildet ist und daß als schalldämmendes Material (25) ein Schaumstoff verwendet wird. Grundsätzlich sind aber auch andere Materialpaarungen denkbar.

Eine Anwendung des Verfahrens und der Vorrichtung kann beispielsweise im Zusammenhang mit Geräten für die CPAP-Therapie oder im Zusammenhang mit anderen Schlaftherapien erfolgen. Ein grundsätzlicher Erfindungsgedanke ist darin zu sehen, daß die unmittelbar luftführenden Teile austauschbar ausgebildet werden.

In den Innenraum (4) können separate Strömungsleitelemente eingesetzt werden, um eine Unterteilung des Innenraumes (4) zur Optimierung der Strömungsführung zu erreichen. Die Strömungsleitelemente können beispielsweise aus einem festen Trägerteil aus Kunststoff ausgebildet sein, auf dem schalldämmendes Material, beispielsweise Schaumstoff fixiert ist. Die Strömungsleitelemente stehen vorzugsweise lose und herausnehmbar innerhalb des Innenraumes (4) und werden lediglich durch Formschluß oder elastische Verspannungen fixiert.

Fig. 9 zeigt eine perspektivische Darstellung eines Strömungsleitelementes (27), das zur Strömungsführung in den Innenraum (4) eingesetzt werden kann. Das Strömungsleitelement (27) besteht typischer Weise aus Kunststoff und kann zur Schalldämmung mit Schaumstoffteilen versehen werden. Es ist eine Strömungsführung (28) eingezeichnet, die veranschaulicht, daß im Bereich eines Eintrittes ein gerundetes Leitelement (29) angeordnet ist. Darüber hinaus weist das Strömungsleitelement (27) im wesentlichen senkrecht relativ zueinander angeordnete Unterteilungswände (30, 31) auf, die eine labyrinthartige Strömungsführung unterstützen. Insbesondere ist daran gedacht, im Bereich eines Überganges von Raumbereichen, die jeweils durch die Unterteilungen (30, 31) voneinander getrennt sind, gerundete Leitelemente (32, 33) anzuordnen. Vorzugsweise ist das Strömungsleitelement (27) mit einer quaderartigen Außenkontur zu versehen.

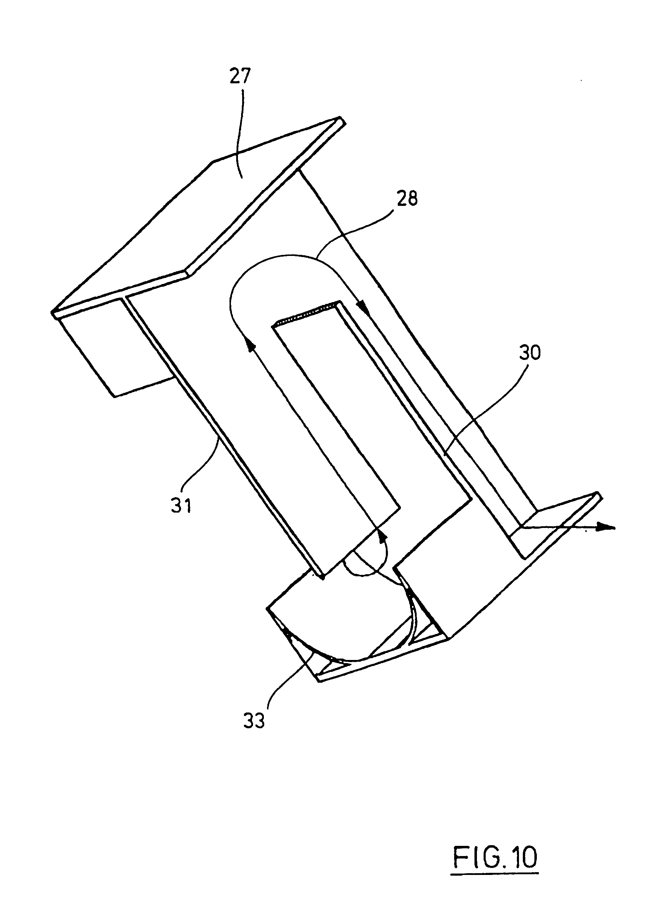

Fig. 10 veranschaulicht die Gestaltung des Strömungsleitelementes (27) im Bereich eines Strömungsaustrittes. Im Bereich dieses Strömungsaustrittes kann auf gerundete Leitelemente verzichtet werden, da eine Überleitung zum Gebläse erfolgt.

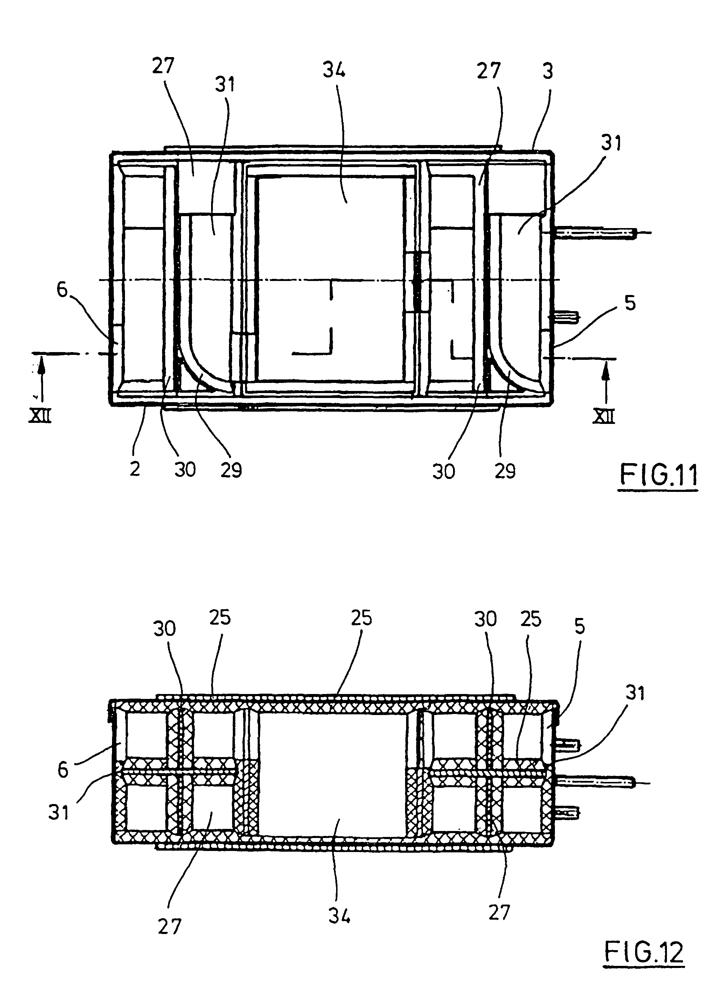

Fig. 11 zeigt das Gehäuseunterteil (2) mit zwei eingesetzten Strömungsleitelementen (27) sowie einem Motorrahmen (34), der zur Aufnahme des Gebläses vorgesehen ist.

Aus der Querschnittdarstellung in Fig. 12 ist erkennbar, wie durch die Strömungsleitelemente (27) eine labyrinthartige Strömungsführung bereitgestellt wird. Insbesondere ist auch erkennbar, wie die Überleitung der Atemgasströmung im den Bereich des Motorrahmens (34) erfolgt. Darüber hinaus ist zu erkennen, daß die in Fig. 9 und Fig. 10 dargestellten Wandungen des Strömungsleitelementes (27) mit schalldämmendem Material (25) versehen sind.

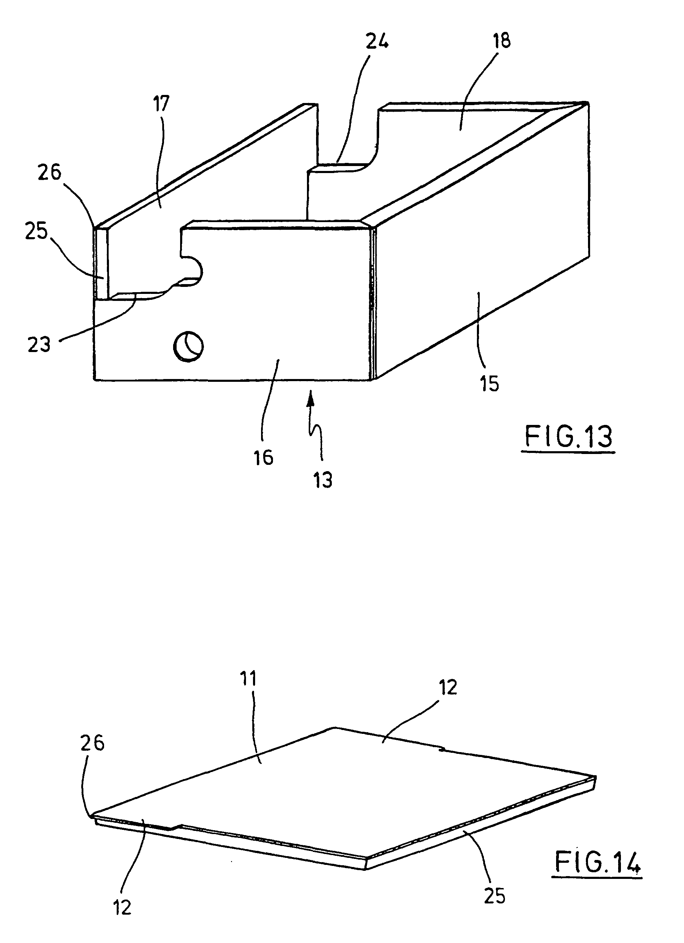

Fig. 13 zeigt das Auskleidungsteil (13) gemäß Fig. 7 nach einem Hochschwenken der Seitenklappen (15, 16, 17, 18), um ein Einfügen des Auskleidungsteiles (13) in das Gehäuseunterteil (2) zu ermöglichen. Insbesondere ist erkennbar, daß die plattenartigen Träger (26) außenseitig und daß das schalldämmende Material (25) innenseitig angeordnet sind.

Fig. 14 zeigt das Einsatzteil (11) in einer perspektivischen Darstellung. Auch hier ist erkennbar, daß in der Zeichenebene der plattenartige Träger (26) oben und das schalldämmende Material (25) unten angeordnet sind. Dies führt dazu, daß nach einem Einfügen des Einsatzteiles (11) in den Gehäusedeckel (7) der plattenartige Träger (26) am Gehäusedeckel (7) anliegt und daß das schalldämmende Material (25) dem Innenraum (4) zugewandt ist.

## Patentansprüche

1. Verfahren zur Schalldämmung bei der Beatmung eines Patienten, bei dem mindestens ein Teil des Atemgases durch eine Vorrichtung nach einem der Ansprüche 8 bis 15 hindurchgeleitet wird, wobei das schalldämmende Material nach einer vorgebbaren Betriebsdauer aus der Dämpfungsbox entnommen wird, und anschließend eine Desinfektion eines Gehäuses der Dämpfungsbox durchgeführt wird und in die Dämpfungsbox ein keimfreies schalldämmendes Material zur Wiederherstellung der Betriebsfähigkeit eingesetzt wird.

2. Verfahren nach Anspruch 1, **dadurch gekennzeichnet, daß** die Desinfektion mindestens zum Teil thermisch durchgeführt wird.

3. Verfahren nach Anspruch 1, **dadurch gekennzeichnet, daß** die Desinfektion mindestens zum Teil chemisch durchgeführt wird.

4. Verfahren nach Anspruch 1, **dadurch gekennzeichnet, daß** die Desinfektion mindestens zum Teil durch Strahlungsbeaufschlagung durchgeführt wird.

5. Verfahren nach einem der Ansprüche 1 bis 4, **dadurch gekennzeichnet, daß** das schalldämmende Material (25) durch Eigenelastizität innerhalb der Dämpfungsbox (1) fixiert wird.

6. Verfahren nach einem der Ansprüche 1 bis 5, **dadurch gekennzeichnet, daß** ein Strömungsweg innerhalb der Dämpfungsbox (1) durch herausnehmbare Strömungsleitelemente begrenzt wird.

7. Verfahren nach einem der Ansprüche 1 bis 6, **dadurch gekennzeichnet, daß** die Atemgasdurchleitung durch die Dämpfungsbox (1) hindurch durch ein innerhalb der Dämpfungsbox (1) angeordnetes Gebläse unterstützt wird.

8. Vorrichtung zur Schalldämmung bei der Beatmung eines Patienten, die als eine Dämpfungsbox (1) ausgebildet ist, die einen von einem Gehäuse umschlossenen Innenraum aufweist, der mit mindestens einem Atemgaseinlaß (5) und mit mindestens einem Atemgasauslaß (6) versehen ist und in der mindestens bereichsweise ein schalldämmendes Material (25) zur Begrenzung mindestens eines Strömungskanals eingesetzt ist, **dadurch gekennzeichnet, daß** das schalldämmende Material (25) plattenartig gestaltet und lose in das Gehäuse eingesetzt ist und daß das schalldämmende Material (25) durch elastische Verspannungen innerhalb des Gehäuses fixiert ist sowie daß das schalldämmende Material (25) auf einen plattenartig gestalteten Träger (26) aufgebracht ist.

9. Vorrichtung nach Anspruch 8, **dadurch gekennzeichnet, daß** das schalldämmende Material (25) aus Schaumstoff ausgebildet ist.

10. Vorrichtung nach Anspruch 8 oder 9, **dadurch gekennzeichnet, daß** mindestens ein Teil des schalldämmenden Materials (25) als ein Einsatzteil (11) für einen Gehäusedeckel (7) der Dämpfungsbox (1) ausgebildet ist.

11. Vorrichtung nach Anspruch 8 oder 9, **dadurch gekennzeichnet, daß** mindestens ein Teil des schalldämmenden Materials (25) als ein Auskleidungsteil (13) für ein Gehäuseunterteil (2) der Dämpfungsbox (1) ausgebildet ist.

12. Vorrichtung nach Anspruch 11, **dadurch gekennzeichnet, daß** das Auskleidungsteil (13) einteilig als Faltbauelement mit Falzungen (19, 20, 21, 22) ausgebildet ist.

13. Vorrichtung nach Anspruch 11 oder 12, **dadurch gekennzeichnet, daß** das Auskleidungsteil (13) ein Zentralelement (14) sowie Seitenklappen (15, 16, 17, 18) aufweist.

14. Vorrichtung nach einem der Ansprüche 8 bis 13, **dadurch gekennzeichnet, daß** in den Innenraum (4) Strömungsleitelemente einsetzbar sind.

15. Vorrichtung nach einem der Ansprüche 8 bis 14, **dadurch gekennzeichnet, daß** im Innenraum (4) ein Gebläse angeordnet ist.

## Claims

1. Method of absorbing sound during the artificial respiration of a patient, with which method at least some of the respiratory gas is passed through an apparatus according to one of claims 8 to 15, wherein the sound-absorbing material after a predefinable operating period is removed from the damping box, and then disinfection of a housing of the damping box is effected and a sterile sound-absorbing material is inserted into the damping box to restore the operating ability.

2. Method according to claim 1, **characterized in that** the disinfection is at least partly effected thermally.

3. Method according to claim 1, **characterized in that** the disinfection is at least partly effected chemically.

4. Method according to claim 1, **characterized in that** the disinfection is at least partly effected by irradiation.

5. Method according to one of claims 1 to 4, **characterized in that** the sound-absorbing material (25) is fixed by inherent elasticity inside the damping box (1).

6. Method according to one of claims 1 to 5, **characterized in that** a flow path is delimited inside the damping box (1) by means of removable flow baffle elements.

7. Method according to one of claims 1 to 6, **characterized in that** conveying of the respiratory gas through the damping box (1) is assisted by means of a blower disposed inside the damping box (1).

8. Apparatus for absorbing sound during the artificial respiration of a patient, which apparatus is designed as a damping box (1) having an interior, which is enclosed by a housing and provided with at least one respiratory gas inlet (5) and with at least one respiratory gas outlet (6) and in which there is inserted at least in sections a sound-absorbing material (25) for delimiting at least one flow channel, **characterized in that** the sound-absorbing material (25) is of a plate-like configuration and is inserted loosely into the housing and that the sound-absorbing material (25) is fixed by elastic bracing inside the housing and that the sound-absorbing material (25) is applied onto a substrate (26) of a plate-like configuration.

9. Apparatus according to claim 8, **characterized in that** the sound-absorbing material (25) is formed from foamed material.

10. Apparatus according to claim 8 or 9, **characterized in that** at least some of the sound-absorbing material (25) is designed as an insert part (11) for a housing lid (7) of the damping box (1).

11. Apparatus according to claim 8 or 9, **characterized in that** at least some of the sound-absorbing material (25) is designed as a lining part (13) for a housing bottom part (2) of the damping box (1).

12. Apparatus according to claim 11, **characterized in that** the lining part (13) is designed in one piece as a folding structural element having folds (19, 20, 21, 22).

13. Apparatus according to claim 11 or 12, **characterized in that** the lining part (13) comprises a central element (14) as well as side flaps (15, 16, 17, 18).

14. Apparatus according to one of claims 8 to 13, **characterized in that** flow baffle elements are insertable into the interior (4).

15. Apparatus according to one of claims 8 to 14, **characterized in that** a blower is disposed in the interior (4).

## Revendications

1. Procédé d'isolation acoustique de la respiration d'un patient, dans lequel au moins une partie du gaz respiratoire est passée à travers un dispositif selon l'une des revendications 8 à 15, le matériau d'isolation acoustique étant enlevé de la boîte d'isolation après une durée d'utilisation prédéterminée, une désinfection du boîtier de la boîte d'isolation étant ensuite réalisée et un matériau d'isolation acoustique exempt de germes étant placé dans la boîte d'isolation pour rétablir sa capacité de fonctionnement.

2. Procédé selon la revendication 1, **caractérisé en ce que** la désinfection est réalisée au moins en partie thermiquement.

3. Procédé selon la revendication 1, **caractérisé en ce que** la désinfection est réalisée au moins en partie chimiquement.

4. Procédé selon la revendication 1, **caractérisé en ce que** la désinfection est réalisée au moins en partie par irradiation par un rayonnement.

5. Procédé selon l'une des revendications 1 à 4, **caractérisé en ce que** le matériau (25) d'isolation acoustique est fixé à l'intérieur de la boîte d'isolation (1) par son élasticité intrinsèque.

6. Procédé selon l'une des revendications 1 à 5, **caractérisé en ce qu'**un parcours de circulation dans la boîte d'isolation (1) est délimité par des éléments amovibles de guidage de la circulation.

7. Procédé selon l'une des revendications 1 à 6, **caractérisé en ce que** le débit du gaz respiratoire dans la boîte d'isolation (1) est soutenu par un ventilateur disposé à l'intérieur de la boîte d'isolation (1).

8. Dispositif d'isolation acoustique de la respiration d'un patient, qui est configuré comme une boîte d'isolation (1) qui présente un espace intérieur entouré par un boîtier doté d'au moins une admission (5) de gaz respiratoire et d'au moins une sortie (6) de gaz respiratoire, et dans au moins certaines parties de laquelle un matériau (25) d'isolation acoustique est placé de manière à délimiter au moins un canal de circulation, **caractérisé en ce que** le matériau (25) d'isolation acoustique est configuré en forme de plaque et est placé de manière lâche dans le boîtier, et **en ce que** le matériau (25) d'isolation acoustique est fixé à l'intérieur du boîtier par contraintes élastiques, et **en ce que** le matériau (25) d'isolation acoustique est appliqué sur un support (26) configuré en plaque.

9. Dispositif selon la revendication 8, **caractérisé en ce que** le matériau (25) d'isolation acoustique est réalisé en mousse.

10. Dispositif selon les revendications 8 ou 9, **caractérisé en ce qu'**au moins une partie du matériau (25) d'isolation acoustique est configurée comme pièce de garniture (11) d'un couvercle (7) du boîtier de la boîte d'isolation (1).

11. Dispositif selon les revendications 8 ou 9, **caractérisé en ce qu'**au moins une partie du matériau (25) d'isolation acoustique est configurée comme pièce de revêtement (13) d'une partie inférieure (2) du boîtier de la boite d'isolation (1).

12. Dispositif selon la revendication 11, **caractérisé en ce que** la pièce d'habillage (13) est configurée en une seule pièce, comme module repliable dotée de plis (19, 20, 21, 22).

13. Dispositif selon la revendication 11 ou 12, **caractérisé en ce que** la pièce d'habillage (13) présente un élément central (14) ainsi que des rabats latéraux (15, 16, 17, 18).

14. Dispositif selon l'une des revendications 8 à 13, **caractérisé en ce que** des éléments de guidage de la circulation peuvent être insérés dans l'espace intérieur (4).

15. Dispositif selon l'une des revendications 8 à 14, **caractérisé en ce qu'**un ventilateur est disposé dans l'espace intérieur (4).
